# EUROPEAN PATENT APPLICATION

(11) **EP 1 964 606 A1**
(43) Date of publication of application: **03.09.2008**
(21) Application number: 08002296.5
(22) Date of filing: 07.02.2008
(51) Int. Cl.: B01J 19/00, C12Q 1/68

(54) **Oligomer probe array**

(30) Priority: 13.02.2007 KR 20070015056
(71) Applicant: Samsung Electronics Co., Ltd., Suwon-si, Gyeonggi-Do (KR)
(72) Inventor: Kim, Won-Sun, Suwon-si Gyeonggi-do (KR); Hah, Jung-Hwan, Toyonaka City Osaka Prefacture, 560-0083 (JP); Chi, Sung-Min, Hwaseong-si Gyeonggi-do (KR); Kim, Kyoung-Seon, Suwon-si Gyeonggi-do (KR); Ryoo, Man-Hyoung No.104-2202 The Sharp Starpark, Seongnam-si Gyeonggi-do (KR)
(74) Representative: Kuhnen & Wacker

(57) **Abstract**

An oligomer probe array (100) includes a substrate (110), an immobilization layer (120) on the substrate, nanoparticles (140) coupled to the immobilization layer and forming a photonic crystal structure, and oligomer probes (165) coupled to the nanoparticles. The immobilization layer (120) includes probe cell regions (A) coupled to nanoparticles. Adjacent probe cell regions are separated by a probe cell separation region (B).

## Description

### BACKGROUND OF THE INVENTION

### 1. Field of the Invention

The present invention relates to an oligomer probe array, and a method of fabricating the same, and more particularly, the present invention relates to an oligomer probe array having probe cell regions coupled to nanoparticles.

### 2. Description of the Related Art

As a result of the genome project, genome nucleotide sequences of a variety of organisms have been found, which has increased interest in using oligomer probe arrays of biopolymer microchips. The oligomer probe array is extensively used to perform gene expression profiling and genotyping to detect mutation and polymorphism, such as single nucleotide polymorphism (SNP), to analyze protein and peptide, to perform screening of potential drugs and to develop and fabricate new drugs.

In order to develop an oligomer probe array, it is important to realize the efficiency of the molecular interface between biomaterials and semiconductors, such as silicon, and to effectively use the intrinsic functions of the biomaterials. Particularly, in oligomer probe arrays, such as DNA chips or protein chips, related biomaterials are immobilized in a predetermined region on a micrometer scale.

The type of genetic information, ranging from genes to nucleotides, which are minimum constituent units of DNA, may be analyzed using the oligomer probe array.
Accordingly, due to the rule of design, the probe cell has been reduced from tens of micrometers to a few micrometers. Therefore, there is a demand for a highly integrated oligomer probe array for improving the reaction yield.

Also, improved detection sensitivity after hybridization with a target sample using an oligomer probe array is needed. In the case of the conventional oligomer probe array, target samples (for example, DNA) are labeled with fluorescent materials and hybridized with oligomer probes on the oligomer probe array. Using a confocal microscope or a CCD camera, signals emitted from the residual fluorescent material on the oligomer probe array are detected.

In the case of using a confocal microscope in optical detection methods, resolution is excellent, but the detection time is long. In comparison, when using a CCD camera, the detection rate is fast, but the resolution is comparatively poor. Accordingly, in order to use a CCD type scanner rather than a confocal type scanner upon detecting hybridization signals, various studies are in progress for increasing the amount of fluorescent material attached to target DNA.

In the above optical detection methods, a small portion of the hybridization signals is not detected, and it is difficult to detect hybridization signals accurately due to signal to noise ratios (SNR) generated around spots. For example, amplifying a signal using biotin-streptavidin increases the number of fluorescence labels to improve the fluorescence intensity, but noise may be increased in the chemical reaction.

### SUMMARY OF THE INVENTION

According to an aspect of the present invention, there is provided an oligomer probe array including a substrate, an immobilization layer on the substrate, nanoparticles coupled to the immobilization layer and forming a photonic crystal structure, and oligomer probes coupled to the nanoparticles. The immobilization layer includes probe cell regions coupled to nanoparticles. Adjacent probe cell regions are separated by a probe cell separation region.

According to another aspect of the present invention, there is provided a method of fabricating an oligomer probe array. The method includes forming an immobilization layer on a surface of a substrate, the immobilization layer including multiple probe cell regions; coupling nanoparticles to the probe cell regions of the immobilization layer to form a photonic crystal structure; and coupling oligomer probes to the nanoparticles. Adjacent probe cell regions are separated by a probe cell separation region.

### BRIEF DESCRIPTION OF THE DRAWINGS

The embodiments of the present invention will be described with reference to the attached drawings, in which:

FIG. 1A is a layout of a probe cell region of the oligomer probe array, according to a first embodiment of the present invention;

FIG. 1B is a sectional view of the oligomer probe array, according to the first embodiment of the present invention;

FIGS. 1C and 1D are partial plan views of probe cell regions illustrating a photonic crystal structure formed by nanoparticles, according to the first embodiment of the present invention;

FIG. 1E is a sectional view of the oligomer probe array coupling with target molecules labeled with fluorescent material, according to the first embodiment of the present invention;

FIG. 1F is a sectional view of the oligomer probe array, according to a second embodiment of the present invention;

FIGS. 2A and 2B are sectional views of the oligomer probe arrays, according to third and fourth embodiments of the present invention;

FIG. 3A is a layout of a probe cell region of the oligomer probe arrays, according to a fifth embodiment of the present invention;

FIG. 3B is a sectional view of the oligomer probe array, according to the fifth embodiment of the present invention;

FIG. 4A is a layout of a probe cell region of the oligomer probe arrays, according to a sixth embodiment of the present invention;

FIGS. 4B and 4D are sectional views of the oligomer probe arrays, according to sixth to eighth embodiments of the present invention;

FIGS. 5A to 5I and 6 are cross-sectional views of intermediate structures during a fabrication process of the oligomer probe array, according to the first embodiment of the present invention;

FIGS. 7A to 7D are cross-sectional views of intermediate structures during a fabrication process of the oligomer probe array, according to the third embodiment of the present invention;

FIGS. 8A to 8E are cross-sectional views of intermediate structures during a fabrication process of the oligomer probe array, according to the fifth embodiment of the present invention;

FIGS 9A to 9B are cross-sectional views of intermediate structures during a fabrication process of the oligomer probe array, according to the sixth embodiment of the present invention;

FIGS. 10A to 10B are cross-sectional views of intermediate structures during a fabrication process of the oligomer probe array, according to the eighth embodiment of the present invention; and

FIG. 11 is a graph illustrating fluorescence intensity measured after hybridization between an oligomer probe array and DNA.

### DESCRIPTION OF THE EMBODIMENTS

The present invention will now be described more fully in the following detailed description with reference to the accompanying drawings, in which exemplary embodiments of the invention are shown. The invention, however, may be embodied in various different forms, and should not be construed as being limited only to the illustrated embodiments. Rather, these embodiments are provided as examples, to convey the concept of the invention to one skilled in the art. Accordingly, known processes, elements, and techniques are not described with respect to some of the embodiments of the present invention.

The terminology used herein is for the purpose of describing particular embodiments only and is not intended to be limiting of the invention. As used herein, singular forms are intended to include plural forms as well, unless the context clearly indicates otherwise. It will be further understood that the terms "comprises" and/or "comprising" specify the presence of stated features, integers, steps, operations, elements, and/or components, but do not preclude the presence or addition of one or more other features, integers, steps, operations, elements, components, and/or groups thereof. As used herein, the term "and/or" includes any and all combinations of one or more of the associated listed items. Throughout the drawings and written description, like reference numerals will be used to refer to like or similar elements.

Furthermore, the present invention will be described, in part, with reference to cross-sectional views and/or schematic views depicting various embodiments of the invention. It is understood, however, that the profile of an exemplary view may be modified according to manufacturing techniques, tolerances and/or allowances. Thus, the depicted embodiments of the invention are not intended to limit the scope of the present invention, but cover all alterations and modifications that may result from variations in the manufacturing processes. In addition, in each drawing of the present invention, constituent elements are not necessarily drawn to scale, and may be exaggerated or contracted for the convenience of description.

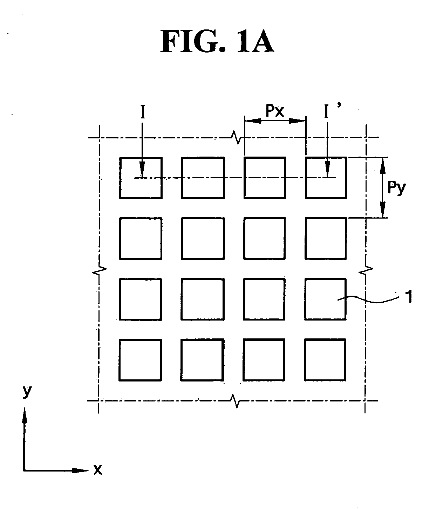

An oligomer probe array according to embodiments of the present invention will be described in detail with reference to the accompanying drawings. First, an oligomer probe array 100 according to a first exemplary embodiment of the present invention will be described in detail with reference to FIGS. 1A to 1E.

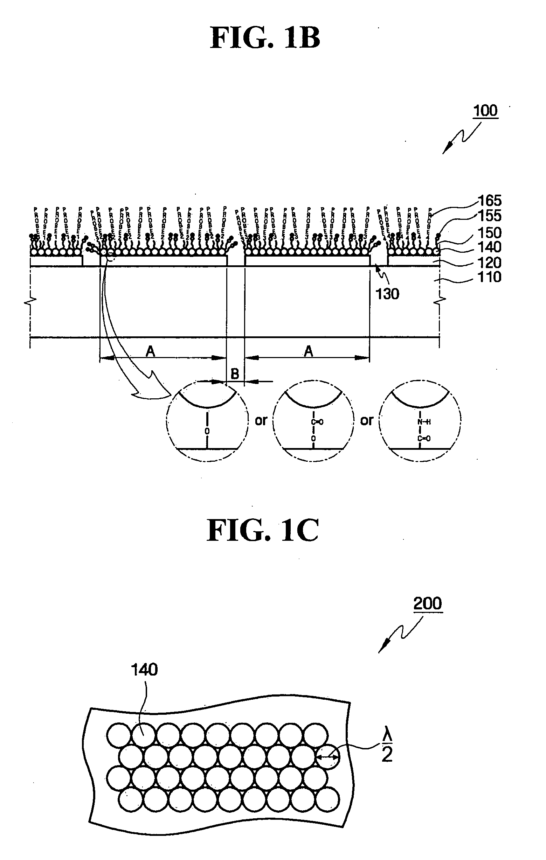

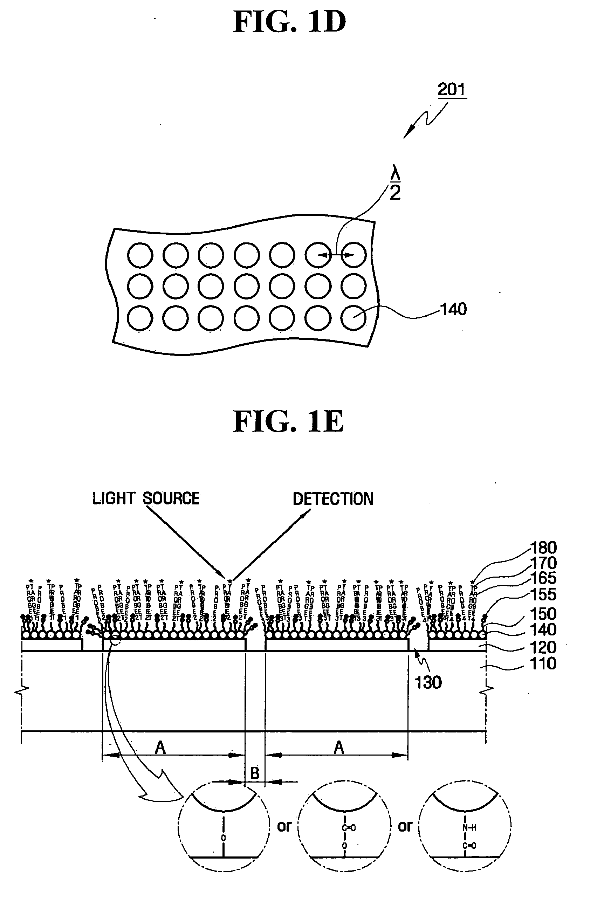

FIG 1A is a layout of a probe cell region of an oligomer probe array according to the first embodiment of the present invention, and FIG. 1B is a cross-sectional view of the oligomer probe array taken along the line A-A' shown in FIG 1A. FIGS. 1C and 1D are partial plan views of probe cell regions illustrating a photonic crystal structure formed by other nanoparticles according to the first embodiment of the present invention. FIG 1E is a sectional view of an oligomer probe array coupling with target molecules labeled with fluorescent material, according to the first embodiment of the present invention.

An oligomer probe array 100 according to the first embodiment of the present invention includes a substrate 110, an immobilization layer 120 on the substrate 110, nanoparticles 140 coupled to the immobilization layer 120 and forming photonic crystal structures 200, 201 and oligomer probes 165 coupled with the nanoparticles 140. The immobilization layer 120 includes multiple probe cell regions A coupled with corresponding sets of nanoparticles 140. The probe cell regions A are separated from one another by a probe cell separation region B.

With reference to FIG. 1A, multiple probe cell region patterns 1 are arranged in row and column directions to form a matrix. Specifically, probe cell region patterns 1 are each arranged in a first pitch Px and a second pitch Py along X and Y directions, respectively. In FIG. 1A, the first pitch Px and the second pitch Py are shown as being equal for purposes of explanation, although the pitches may be different, if necessary, without departing from the spirit and scope of the present invention.

First, the substrate 110 is described. The substrate 110 may be a flexible or rigid substrate. Examples of the flexible substrate include membranes or plastic films made of nylon or nitrocellulose. Examples of the rigid substrate include a silicon substrate or a transparent glass substrate made of soda-lime glass. In the case of the silicon substrate or the transparent glass substrate, nonspecific bonding is minimized during a hybridization process. Additionally, the silicon substrate or the transparent glass substrate is advantageous in that the process of fabricating various thin films or the photolithography process typically used to fabricate semiconductor devices or LCD panels can be applied without modification.

Next, an immobilization layer 120 is described. The immobilization layer 120 includes multiple probe cell regions A coupled with sets of nanoparticles 140. Adjacent probe cell regions A are separated by probe cell separation regions B. Each probe cell region A of the immobilization layer 120 includes a functional group allowing nanoparticles 140 to couple on its surface.

Oligomer probes 165 having the same sequences can be coupled to a separate probe cell region A mediated by the corresponding nanoparticles 140. The nanoparticles 140 coupled with the oligomer probes 165 having different sequences may be present in a different probe cell region A. The functional groups remaining on the surface of each probe cell region A may be inactived by capping groups 155. The capping groups 155 may include acetylation, for example.

In an embodiment, the immobilization layer 120 is not hydrolyzed, but is substantially stable under a hybridization analysis condition, for example, when the immobilization layer 120 comes into contact with a phosphoric acid having a pH in the range of 6 to 9 or a TRIS buffer. For example, the immobilization layer 120 may be formed from a silicon oxide film (e.g., a PE-TEOS film, an HDP oxide film, a P-SiH₄ oxide film and a thermal oxide film), a silicate (e.g., hafnium silicate and zirconium silicate), a metal oxynitride film (e.g., a silicon oxynitride film, a hafnium oxynitride film and a zirconium oxynitride film), a metal oxide film (e.g., a titanium oxide film, a tantalum oxide film, an aluminum oxide film, a hafnium oxide film, a zirconium oxide film and an indium tin oxide (ITO) film), polyimides, polyamines, metals (e.g., gold, silver, copper and palladium), and/or a polymer (e.g., polystyrene, a polyacrylic acid and polyvinyl). Within the fabrication process, the most stable material should be used when fabricating the semiconductors or the LCDS.

The nanoparticles 140 are coupled to a respective probe cell region A of the immobilization layer 120. The materials forming the immobilization layer 120 and a functional group formed on the surface of the nanoparticles 140 are described.

When the immobilization layer 120 is formed from the silicon oxide film, the silicate or the silicon oxynitride film, for example, the coupling groups of the nanoparticles 140 may include silicon groups that are reacted with Si(OH) groups on the surface of the immobilization layer 120 to form siloxane (Si-O) bonds. For example, the coupling groups may include a -Si(OMe)₃, -SiMe(OMe)₂, -SiMeCl₂, -SiMe(OEt)₂, -SiCl₃, or -Si(OEt)₃ group. Examples of material having capable of forming the siloxane bond include N-(3-(triethoxysilyl)-propyl)-4-hydroxybutyramide, N,N-bis(hydroxyethyl) aminopropyltriethoxysilane, acetoxypropyl-triethoxysilane, 3-glycidoxy propyltrimethoxysilane, and the silicon compound disclosed, for example, in International Patent Application Publication No. WO 00/21967, the contents of which are hereby incorporated by reference.

When the immobilization layer 120 is formed from a metal oxide film, the coupling groups of the nanoparticles 140 may include metal alkoxide or metal carboxylate groups, for example. When the immobilization layer 120 is formed from a silicon nitride film, a silicon oxynitride film, a metal oxynitride film, polyimides or polyamines, the coupling groups of the nanoparticles 140 may include anhydride, hydrochloric acid, alkyl halide or chlorocarbonate groups, for example. When the immobilization layer 120 is formed from metal, the coupling groups of the nanoparticles 140 may include sulfide, selenide, arsenide, telluride or antimonide groups, for example. When the immobilization layer 120 is formed from a polymer, the coupling groups of the nanoparticles 140 may include acryl, styryl, or vinyl groups.

The probe cell separation region B includes an inactive region 130 which surrounds each probe cell region A, and which is not coupled with the nanoparticles 140. In the oligomer probe array 100, according to an embodiment of the present invention, the surface of the substrate 110 is directly exposed in the probe cell separation region B. As a result, since it is possible to prevent the undesired coupling of the nanoparticles 140, the undesired noise resulting from the undesired coupling of the oligomer probes 165 rarely occurs.

Nanoparticles 140 coupled with a probe cell region A of the immobilization layer 120 are now described. The nanoparticles 140 may be substantially in the range of nanometers in terms of size. The nanoparticles 140 provide a spatial margin for hybridization between oligomer probes 165 and target molecules 170 (FIG. 1E), and form photonic crystal structures 200, 201 to amplify the wavelength of a fluorescent material 180, which is attached to the target molecules 170 hybridized with oligomer probes 165.

The nanoparticles 140 can function as a linker or a spacer, which provides a spatial margin for hybridization between the oligomer probes 165 and the target molecules 170. The oligomer probes 165 can be directly coupled to the immobilization layer 120. However, the coupling of the oligomer probes 165 may be mediated by the nanoparticles 140, making it easier to hybridize with the target molecules 170.

Each of the nanoparticles 140 has a functional group capable of coupling to a probe cell region A, a functional group capable of coupling to a linker 150 when the linker 150 mediates coupling, and a functional group capable of coupling to an oligomer probe 165 when the linker 150 does not mediate coupling, or the like, on its surfaces. The three functional groups may be identical, although they may be different in alternative embodiments. Examples of functional groups include a hydroxyl group, a carboxylic group, an amine group, a halo group or a sulfonate group, although the disclosed embodiments are not limited to these examples.

The coupling of the nanoparticles 140 and probe cell regions A may be a covalent bond of the chemical bond. Therefore, the nanoparticles 140 may be fixed to the immobilization layer 120, that is, the probe cell region A uses a stronger bond in comparison with an electrostatic force or a capillary force. When the nanoparticles 140 are coupled, it is apparent to those skilled in the art that the functional groups of the nanoparticles 140 arer converted into ether bonds, ester bonds or peptide amide bonds. The functional groups which remain on the surface of the nanoparticles 140 may be inactively capped by the capping groups 155. The capping groups 155 may be formed from acetylation, for example.

The nanoparticles 140 can themselves function as linkers, and can also be coupled with the oligomer probes 165 through other linkers 150 or spacers. In this case, the linkers 150 or the spacers contain functional groups capable of being coupled with both nanoparticles 140 and the oligomer probes 165. One end of a linker 150 or a spacer is coupled with a nanoparticle 140 and another end of the linker 150 or the spacer is coupled with an oligomer probe 165. The linkers 150 may work to provide a spatial margin necessary for interaction between an oligomer probe array 100 and a target sample, for example, for hybridization. Accordingly, the length of a linker 150 may be, for example, about 4 to about 50 atoms. If a spatial margin for hybridization is sufficiently maintained, the linkers 150 are not necessary.

The oligonucleic acid may be used as the linkers 150. The oligonucleic acid is used to assure the spatial margin, so that the nucleic acid of about 1 to about 5 mer firstly coupled with the functional groups of the nanoparticles 140 is not hybridized. The length of the oligonucleic acid used as the linkers 150 is not limited to the range, and may fall of the range of about 1 to about 5 mer, without departing from the spirit and scope of the present invention.

The functional groups which remain on the surfaces of the linkers 150 may be inactively capped by the capping groups 155. After the desired coupling is finished, the remaining functional groups are inactivated by the capping groups 155. The capping groups 155 may be formed from acetylation, for example.

The nanoparticles 140 are formed on each probe cell region A to form a photonic crystal structure, such as photonic crystal structures 200 and 201. Each of the photonic crystal structures 200, 201 is referred to as a structure, in that two or more materials having different refractive indexes or dielectric constants are arranged in a predetermined fashion to form a photonic band gap to prevent an electromagnetic wave from entering the photonic crystal structures 200, 201. The photonic crystal structures 200, 201 form a photonic band gap and can function as an optical filter, which reflects or transmits light at a specific wavelength to amplify or reduce the gap.

The photonic crystal structures 200, 201 are formed from the nanoparticles 140. Although spherical nanoparticles 140 are illustrated in the drawings (FIGS. 1C and 1D), the embodiments of the invention are not limited to nanoparaticles having spherical shapes. That is, the nanoparticles 140 may have various different shapes, such as oval, wedge and stripe shapes, without departing from the spirit and scope of the present invention. Also, the photonic crystal structures 200, 201 formed from the nanoparticles 140 may have a face-centered cubic structure, a diamond-like structure, an inverse opal structure or the like.

A principle of light transmission in the photonic crystal structures 200, 201 is similar to the way in which X-rays are diffracted by periodicity, originated with the Bragg condition (i.e., dsinθ=nλ/2, where θ is an angle of incidence, λ is a wavelength, d is a distance between atoms, and n is an integer). That is, when light meets with the photonic crystal structures 200, 201, light within a specific wavelength region is not transmitted and is reflected on the surface of a first dielectric, and light with a different wavelength region is transmitted and is reflected on a second dielectric in the photonic crystal structure 200, 201. At this time, the wavelength region of the light not transmitted is referred to as a photonic band gap. In general, the photonic crystal structures 200, 201 increase the photonic band gap as a refractive index difference between the first dielectric and the second dielectric is larger. In other words, the light controllable region becomes be wider. Air may be used as the two dielectrics provided between the sets of nanoparticles 140.

The energy band gap of the photonic crystal structures 200, 201 varies depending on the size of the nanoparticles 140 and a dielectric constant. Examples of the nanoparticles 140 include spherical polystyrene, silica, glass, magnet, Wang resin, Merrifield resin, metal, plastic, cellulose, sephadex or sepharose, but are not limited thereto. The nanoparticles 140 having a dielectric constant between about 1 through about 16 can be used. The nanoparticles 140 can include, for example, an inorganic substance or an organic polymer. Here, examples of inorganic substances include silicon, polysilicon, metal, silicon oxide, silicon nitride, and examples of organic polymers include polymethyl methacrylate, polystyrene, polydimethyl siloxane.

As shown in FIG. 1C, the photonic crystal structure 200 formed by nanoparticles 140 may be a self-assembled monolayer. In this case, the distance between the centers of adjacent nanoparticles 140 is the same as the diameter of the nanoparticles 140. Therefore, the diameter of the nanoparticles 140 can be λ/2 (λ is the wavelength of detected fluorescent light). For example, nanoparticles 140 are spherical particles, and their diameters can be in the range of about 100 nm to about 1000 nm, preferably in the range of about 200 nm to about 300 nm.

As shown FIG. 1D, the photonic crystal structure 201 is formed by nanoparticles 140 arranged in predetermined intervals. In this case, the distance between the centers of adjacent nanoparticles 140 can be λ/2 (λ is the wavelength of detected fluorescent light). For example, the distance between the centers of adjacent nanoparticles can be in the range of about 100 nm to about 1000 nm, preferably in the range of about 200 nm to about 300 nm.

Next, the oligomer probes 165 coupled with the nanoparticles 140 are described. Each of the oligomer probes 165 is a polymer that is formed of two or more monomers covalently bonded to each other, and has a molecular weight of about 1000 or less. However, the molecular weight is not limited to this value. The oligomer may include about 2 to about 500 monomers, and preferably about 5 to about 30 monomers. Examples of the monomers include nucleosides, nucleotides, amino acids or peptides, according to the type of probe fixed to the oligomer probe array.

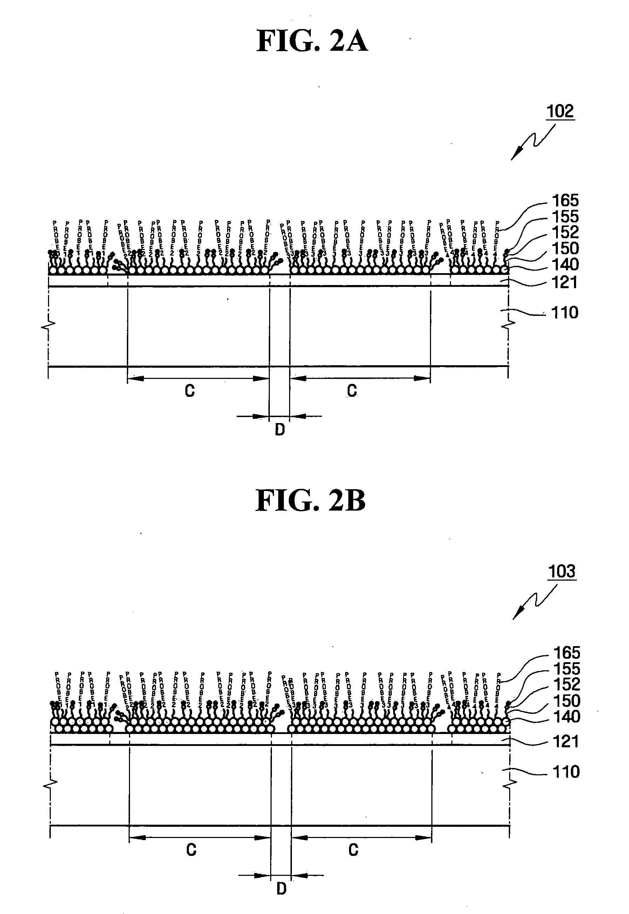

When linkers 150 are interposed, the oligomer probes 165 are coupled with functional groups 152 (FIGS. 2A and 2B) of the linkers 150, and the linkers 150 are connected through nanoparticles 140 to the immobilization layer 120 in the probe cell region A. When linkers 150 are not interposed, the oligomer probes 165 are directly coupled to the nanoparticles 140, and the nanoparticles 140 are fixed to the immobilization layer 120 in the probe cell region A.

Nucleosides and nucleotides may include a known purine or pyrimidine base, or include methylated purine or pyrimidine, or acylated purine or pyrimidine. Furthermore, nucleosides and nucleotides may include known ribose or deoxyribose saccharides, or include modified saccharides in which one or more hydroxyl groups are substituted by halogen atoms or aliphatics, or to which the functional group, such as ether or amine, is bonded.

The amino acid may be an L-, D-, or nonchiral-type amino acid, found in nature. Alternatively, the amino acid may be a modified amino acid or an analog of the amino acid. The peptide is a compound that is formed by amide bonding between a carboxyl group of an amino acid and an amino group of another amino acid. The oligomer probes 165 may be formed from two or more nucleosides, nucleotides, amino acids, and peptides.

As mentioned above, the oligomer probe array 100 has been described according to the first exemplary embodiment of the present invention. With reference to FIG. 1E, detection intensity is increased by photonic crystal structures 200, 201 formed by the nanoparticles 140 of the oligomer probe array 100, according to the first embodiment.

A target molecule 170 is referred to as a biosample, which is analyzed using an oligomer probe array 100. The examples of the target molecule 170 include cDNA, mRNA, an oligonucleotide and a protein, but are not limited thereto.

A fluorescent material 180 is attached to each target molecule 170 to be used for optical detection after hybridization reaction of the target molecule 170 and the corresponding oligomer probe 165. The fluorescent material 180 can be formed from Rhodamine200, Calcium Green, Cyanine 2, Cyanine 3, Cyanine 5, Magnesium Green, Tetramethyl rhodamine, Fluorescein or the like, but is not limited thereto.

For example, the fluorescent material 180, such as Cyanine 3 and Cyanine 5, can be used to confirm whether a reaction occurs between the target molecule 170 and the oligomer probe 165. For example, if Cyanine 3 is used as the fluorescent material 180 of the target molecule 170, the wavelength of light emitted is approximately 570 nm. Therefore, the photonic crystal structure 200, 201 formed by the first dielectric and the second dielectric has a cycle of approximately 285 nm. Accordingly, in the case where the nanoparticles 140 have a photonic crystal structure 200, as shown in FIG. 1C, their diameter is about 285 nm. In the case where the nanoparticles 140 have a photonic crystal structure 201, as shown in FIG. 1D, the distance between the centers of adjacent nanoparticles 140 may be about 285 nm.

The oligomer probe array 100 can be used for detecting target molecules 170. The target molecules 170 are applied to the oligomer probe array 100 to perform hybridization. The target molecules 170 can be analyzed by detecting the position and intensity of hybridization on the oligomer probe array 100. The intensity of hybridization may be analyzed by an optical method, detecting signals which are emitted from the fluorescent material 180 after hybridizing the target molecules 170 labeled with the corresponding fluorescent material 180 and probes 165. The fluorescent signals from the target molecules 170 are passed through the photonic crystal structures 200, 201 to transmit or reflect light at a specific wavelength without absorption. That is, since the photonic crystal structures 200, 201 have an energy band gap at the specific wavelength, if the fluorescent signals are present in the energy band gap region, the internal reflection efficiency is increased to amplify the fluorescent signals. Subsequently, according to the first embodiment of the present invention, upon detecting the wavelength of light emitted from the fluorescent material 180 using a CCD, a signal amplification using a biotin-streptavidin is not needed. The signal amplification using bioaffinity, such as a biotin-streptavidin requires multiple processing steps, such as reacting and washing, thereby increasing chemical noise. However, the signal amplification using the photonic crystal structure 200, 201 does not generate such chemical noise.

Hereinafter, with reference to the drawings, oligomer probe arrays according to second through sixth exemplary embodiments of the present invention are described. For convenience of explanation, like or similar elements in the drawings are indicated by the same reference numerals, and thus the descriptions of these elements will not be repeated.

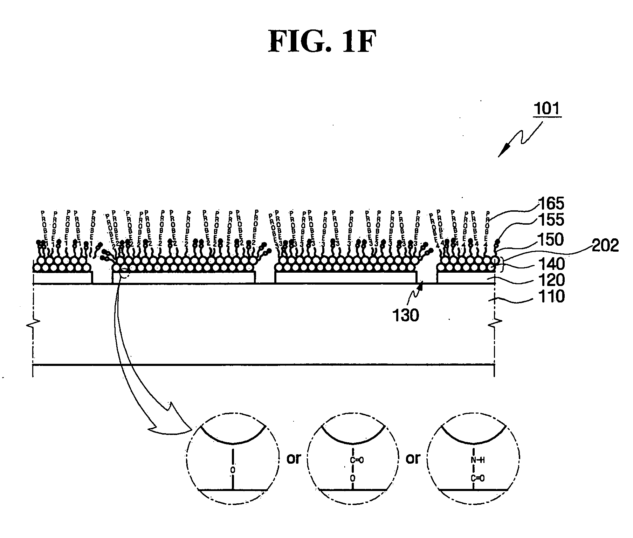

With reference to FIG 1F, an oligomer probe array 101 according to a second embodiment of the present invention will be described. FIG 1F is a sectional view of the oligomer probe array 101, according to the second embodiment.

The oligomer probe array 101 has a two-dimensional photonic crystal structure formed by nanoparticles 140. The oligomer probe array 101 having the two-dimensional photonic crystal structure is able to obtain more accurate signals and to increase detection sensitivity, as compared to the oligomer probe array 100 having a one-dimensional photonic crystal structure. Similarly, the amplification of a signal in a three-dimensional photonic crystal structure (not shown in the drawings) may be larger than that in the two-dimensional photonic crystal structure.

With reference to FIG. 2A, an oligomer probe array 102 according to a third embodiment of the present invention is described. FIG. 2A is a sectional view of the oligomer probe array 102, according to the third embodiment.

More specifically, an immobilization layer 121 formed on the substrate 110 includes multiple activated probe cell regions C coupled with multiple sets of nanoparticles 140. Inactivated regions D surround the activated probe cell regions C and are not coupled with the nanoparticles 140. The activated probe cell regions C may be coupled with the nanoparticles 140.

In terms of functionality, the activated probe cell region C is substantially the same as that described with respect to the probe cell regions A of FIG. 1B, and the inactivated cell region D is the substantially the same as that described with respect to the probe cell separation region B of FIG 1B.

However, since the inactivated cell region D includes the immobilization layer 121, unlike probe cell separation region B and the immobilization layer 120 of FIG 1B, the functional group of the surface of the immobilization layer 121 may be present on the surface of the inactivated region D. Thus, it is necessary to block the action of the functional group. The functional group of the inactivated region D may be inactively capped, for example, by the capping groups 155.

With reference to FIG. 2B, an oligomer probe array 103, according to a fourth embodiment of the present invention, is described. FIG. 2B is a sectional view of the oligomer probe array 103, according to the fourth embodiment.

The oligomer probe array 103 according to the fourth embodiment differs from the oligomer probe array 102 according to the third embodiment in that the oligomer probe array 103 has a two-dimensional photonic crystal structure formed by the nanoparticles 140. The oligomer probe array 103 having the two-dimensional photonic crystal structure is able to obtain more accurate signals and to increase detection sensitivity, as compared to the oligomer probe array 102 having the one-dimensional photonic crystal structure. Likewise, a three-dimensional photonic crystal structure (not shown in the drawings) formed by nanoparticles 140 may have higher detection sensitivity than the two-dimensional photonic crystal structure.

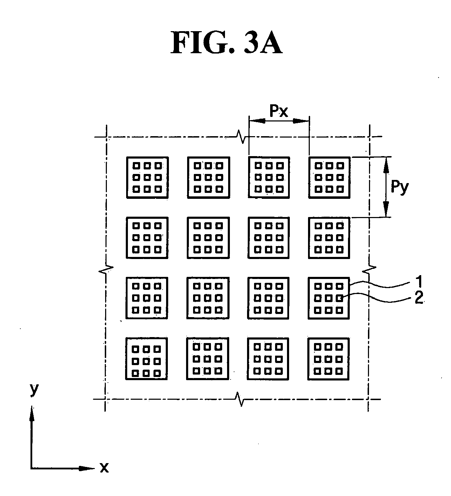

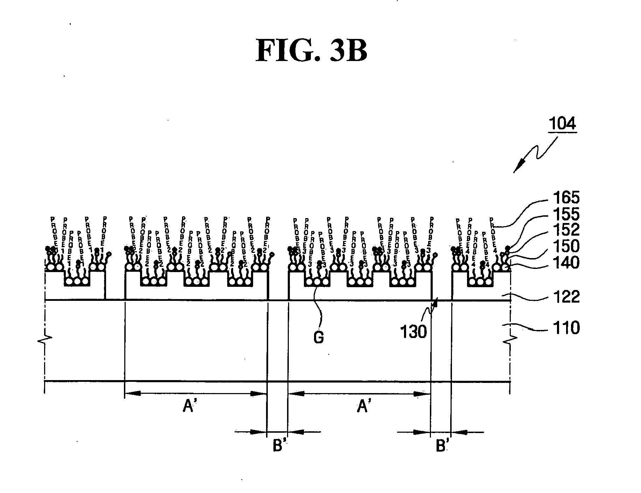

With reference to FIGS. 3A and 3B, an oligomer probe array 104 according to a fifth embodiment of the present invention is described. FIG. 3A is a layout of a probe cell region of the oligomer probe array according to the fifth embodiment, and FIG. 3B is a sectional view of the oligomer probe array according to the fifth embodiment.

With reference to FIG. 3A, multiple probe cell region patterns 1 are arranged in both row and column directions, forming a matrix. Specifically, each of the cell region patterns 1 is arranged in a first pitch Px and a second pitch Py along X and Y directions. In FIG. 3A, the first pitch Px and the second pitch Py are shown as being equal for purposes of explanation, although the pitches may be different, if necessary, without departing from the spirit and scope of the present invention. A multiple groove pattern 2 is arranged in each cell region pattern 1, so that the surface of the cell region pattern 1 is three-dimensional. FIG. 3A illustrates an exemplary groove pattern 2 in the form of regular squares. However, the groove pattern 2 may have various forms, such as rectangles, circles or semicircles, without departing from the spirit and scope of the present invention. Further, the groove pattern 2 may be a unidirectional line pattern that crosses the cell region pattern 1 or a cross line pattern extending in X and Y directions.

FIG. 3B illustrates that the surface of the probe cell region (A') formed on the immobilization layer 122 of the oligomer probe array 104, according to the fifth embodiment, is three-dimensional, which differs from the one-dimensional surface of the probe cell region (A) of the oligomer probe array 100, according to first embodiment. The surface of the probe cell region A' indicates a three-dimensional surface structure as one or more grooves G, formed in the probe cell region A'. However, the various embodiments are not limited to a rectangular groove G as the three-dimensional structure, and other three-dimensional structures may be incorporated. As discussed above, the three-dimensional surface increases the surface area available for coupling with the nanoparticles 140, which in turn increases the area available for coupling with the oligomer probes 165, thereby increasing detection intensity.

The three-dimensional pattern itself of the immobilization layer 122, having one or more groove G, can form a photonic crystal structure (not shown in the drawings). For example, of two dielectrics forming a photonic crystal structure, one can be a dielectric constituting the immobilization layer 122 and the other can be an air. However, in order to form the photonic crystal structure, the pattern of the immobilization layer 122 must be arranged constantly. For example, a width of the groove G by air and the distance between adjacent grooves may be λ/4, respectively.

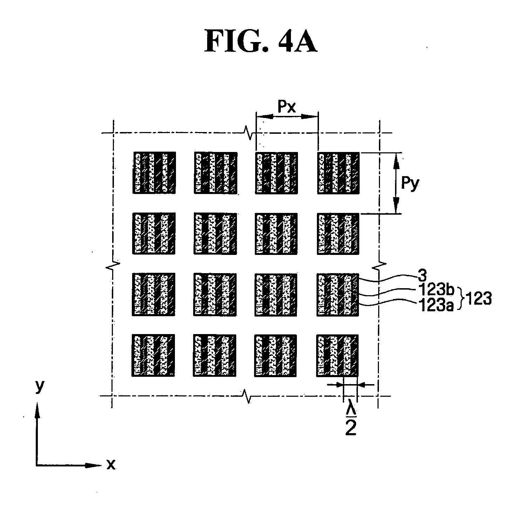

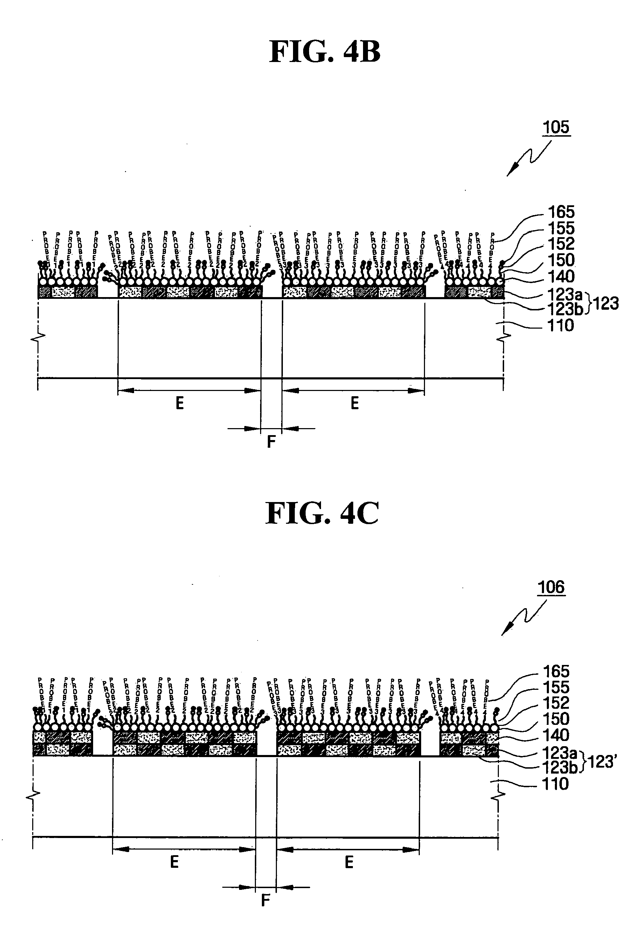

With reference to FIGS. 4A and 4B, an oligomer probe array 105 according to a sixth embodiment of the present invention is described. FIG. 4A is a layout of a probe cell region of the oligomer probe array according to the sixth embodiment, and FIG 4B is a sectional view of the oligomer probe array, according to the sixth embodiment.

With reference to FIG. 4A, multiple probe cell region patterns 3 are arranged in both row and column directions, to form a matrix. Specifically, they are each arranged in a first pitch Px and a second pitch Py along X and Y directions. In FIG. 4A, the first pitch Px and the second pitch Py are shown as being equal for purposes of explanation, although the pitches may be different, if necessary, without departing from the spirit and scope of the present invention. In order to form a photonic crystal structure in cell region patterns 3, two dielectrics 123a, 123b are alternately arranged. The two dielectrics 123a, 123b can be arranged one-dimensionally, but are not limited to this pattern, and can therefore be arranged two-dimensionally or three-dimensionally.

With reference to FIG. 4B, the probe cell region E formed on the immobilization layer 123 of the oligomer probe array 105, according to the sixth embodiment of the present invention, is a photonic crystal structure. Detection sensitivity can be increased by forming photonic crystal structures 200, 201 by nanoparticles 140, as well as an immobilization layer 123.

The immobilization layer 123 on the substrate 110 consists of photonic crystal layers formed with a first dielectric 123a and a second dielectric 123b. The photonic crystal layer is a layer that is arranged by alternating the first dielectric 123a and second dielectric 123b in a first direction, for example, creating the appearance of a striped layer. The one-dimensional photonic crystal structure is constituted as one photonic crystal layer. A width of each of the first dielectric 123a and the second dielectric 123b of the photonic crystal layer may be substantially 1/4 of the emitted light wavelength, for example.

The first dielectric 123a and second dielectric 123b consist of dielectrics having different refractive indexes. The first dielectric 123a and second dielectric 123b consist of, for example, an inorganic substance or an organic polymer. The inorganic substance may include, for example, silicon, polysilicon, metal, silicon oxide, silicon nitride or the like, and the organic polymer may include, fore example, polymethy methacrylate, polystyrene, polydimethyl siloxane or the like.

With reference to FIG 4C, an oligomer probe array 106 according to a seventh embodiment of the present invention is described. FIG. 4C is a sectional view of the oligomer probe array 106, according to the seventh embodiment.

The oligomer probe array 106 according to the seventh embodiment differs from the oligomer probe array 105 according to the sixth embodiment in that the immobilization layer 123 is a two-dimensional photonic crystal structure 123'. The oligomer probe array 106 having the two-dimensional photonic crystal structure can obtain more accurate signals and increase detection sensitivity, as compared to the oligomer probe array 105 having the one-dimensional photonic crystal structure. The first dielectric 123a and second dielectric 123b are arranged alternately in a parallel direction to each other. FIG. 4C illustrates a structure of the immobilization layer 123' having two layers, each having alternating first and second dielectrics. However, embodiments of the invention are not limited thereto, and thus additional photonic crystal layers may be included having alternating first and second dielectrics, without departing from the spirit and scope of the present invention.

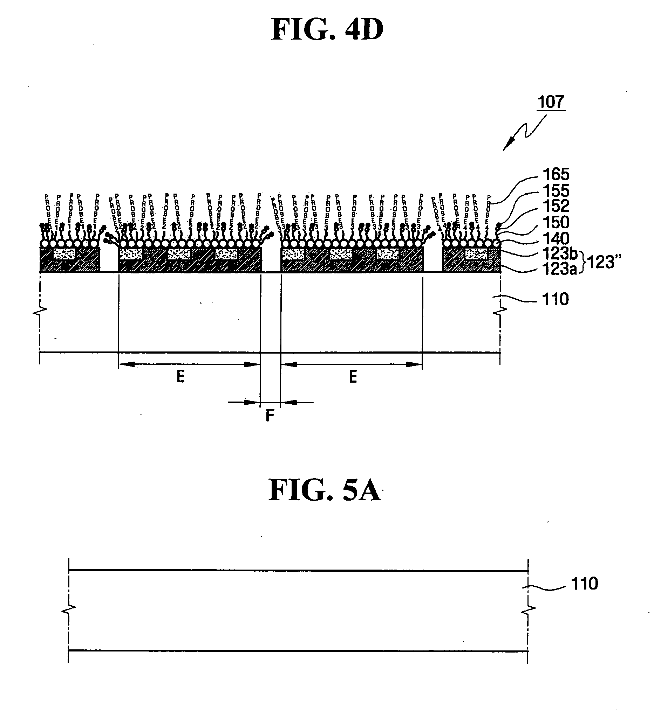

With reference to FIG 4D, an oligomer probe array 107 according to an eighth embodiment of the present invention is described. The immobilization layer 123 is a three-dimensional photonic crystal structure 123", and is substantially the same as the two-dimensional photonic crystal structure described above in that multiple photonic crystal layers are included. However, there is a difference in that an n^{th} photonic crystal layer and an n+1^{st} photonic crystal layer (where n is a natural number) have first dielectrics 123a that are arranged perpendicular to each other, and second dielectrics 123b, alternating between the first dielectrics 123a, that are arranged perpendicular to each other. Accordingly, a next photonic crystal layer is vertically patterned with respect to the preceding photonic crystal layer. Therefore, each dielectric 123a, 123b of each photonic crystal layer is vertically arranged with respect to each other and the same dielectrics of an n^{st} photonic crystal layer and an n+1^{st} photonic crystal layer can be alternately arranged with respect to each other. The three-dimensional photonic crystal structure 123" may have higher detection sensitivity, as compared to the two-dimensional photonic crystal structure 123'.

With reference to FIGS. 5A through 5I and FIGS 1A to 1F, an exemplary method of fabricating an oligomer probe array, according to the first embodiment of the present invention, e.g., as shown in FIG. 1B, is described.

FIGS 5A through 5I are cross-sectional views of intermediate structures at various stages during a fabrication procedure of the oligomer probe array, according to the first embodiment of the present invention.

The method of fabricating the oligomer probe array 100 includes forming an immobilization layer 120 on a substrate 110, coupling nanoparticles 140 to the immobilization layer 120 to form a photonic crystal structure 200, and coupling oligomer probes 165 with the nanoparticles 140. The immobilization layer 120 includes multiple probe cell regions A coupled with the nanoparticles 140, where the probe cell regions A are separated by probe cell separation regions B.

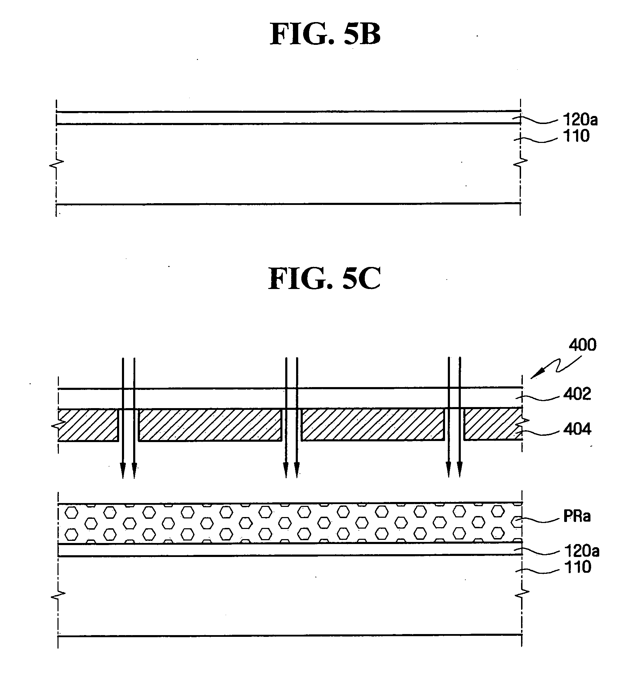

With reference to FIGS. 5A and 5B, a probe cell region forming layer (immobilization layer) 120a is formed on a semiconductor substrate 110. The probe cell region forming layer 120a may be formed, for example, from a silicon oxide layer (such as a PE-TEOS layer, a HDP oxide layer, a P-SiH4 oxide layer or a thermal oxide layer), a silicate (such as a hafnium silicate or a zirconium silicate), a metal oxynitride layer (such as a silicon oxynitride layer, a hafnium oxynitride layer or a zirconium oxynitride layer), a metal oxide layer (such as a titanium oxide layer, a tantalum oxide layer, an aluminum oxide layer, a hafnium oxide layer, a zirconium oxide layer or an ITO), a metal (such as a polyimide, a polyamine, gold, silver, copper or palladium), or a polymer (such as a polystyrene, a poly acrylic acid or a polyvinyl).

The deposition method employed in a semiconductor or LCD fabrication process includes, for example, CVD (Chemical Vapor Deposition), SACVD (SubAtmospheric CVD), LPCVD (Low Pressure CVD), PECVD (Plasma Enhanced CVD), Sputtering, Spin Coating. Materials that can be stably formed on the substrate 110 are used.

With reference to FIG. 5C, a photoresist layer PRa is formed on the probe cell region forming layer 120a. The photoresist layer PRa is exposed to a projection exposure system using a photomask 400, defining a probe cell region. An example of the photomask 400 includes a mask having a checkerboard pattern exposed region by a light shielding pattern 404, defining the probe cell region on a transparent substrate 402. However, the type of light shielding pattern may vary according to the type of photoresist layer being used.

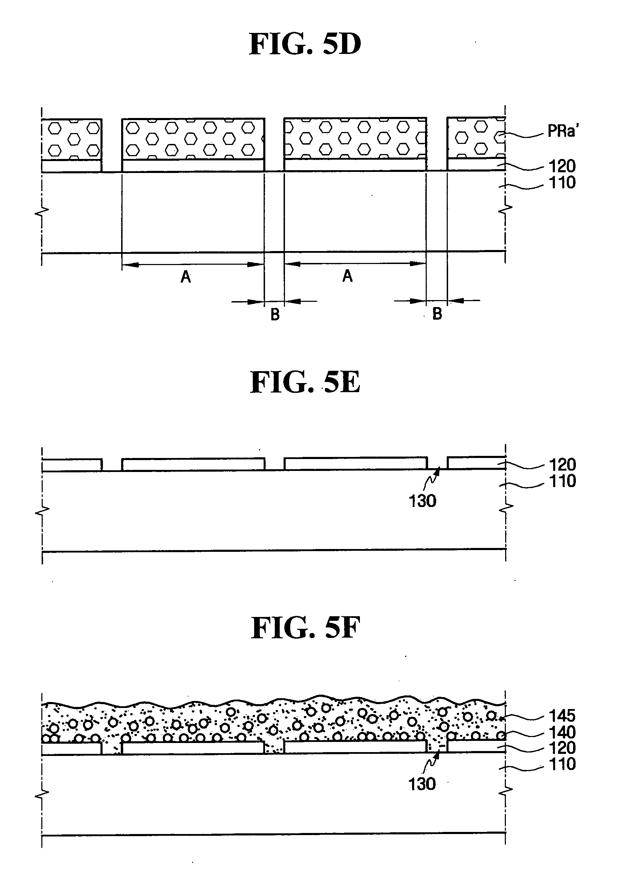

With reference to FIG. 5D, the exposed photoresist layer PRa is exposed to form a photoresist pattern PRa'. The probe cell region forming layer 120a is etched using the photoresist pattern PRa' as an etch mask to form probe cell regions A.

With reference to FIG. 5E, the photoresist pattern PR' is removed, completing fabrication of the probe cell regions A. The structure includes the immobilization layer 120 separated by inactive regions 130, corresponding to probe cell separation regions B located between the probe cell regions A. When a probe cell region A is made of a silicon oxide layer, for example, a SiOH group capable of coupling with an oligomer probe is exposed on the surface of the probe cell region A.

As shown in FIG. 5F, a suspension solution 145 containing nanoparticles 140 is dispersed on the probe cell regions A. Examples of the nanoparticles 140 may include polystyrene beads, and examples of the dispersion may include spin coating.

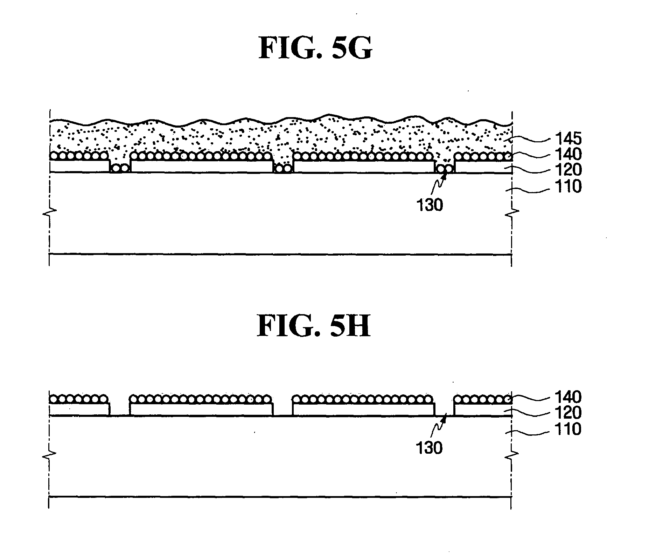

As shown in as shown in FIG. 5G, the nanoparticles 140 are arranged on the probe cell region A following the dispersion. The particles may be arranged to form a self assembled single molecular film on the surfaces of the probe cell regions A. The functional groups that are present in the nanoparticles 140 are coupled with the probe cell regions A. The nanoparticles 140 may be present in the probe cell separation regions B (i.e., the inactive regions 130).

With reference to FIG. 5H, washing and curing processes may be performed to arrange the nanoparticles 140 only on the probe cell regions A. For example, since there are no functional groups capable of being coupled with the nanoparticles 140 in the probe cell separation regions B, the nanoparticles 140 are removed from the probe cell separation regions B.

A photonic crystal structure is formed by the arrangement of nanoparticles 140. As shown in FIGS. 1C and 1D, photonic crystal structures 200, 201 are referred to as structures in which two or more materials having different refractive indexes or dielectric constants are regularly arranged to form a photonic band gap to prevent an electromagnetic wave from entering the inside of the photonic crystal structures 200, 201.

A photonic crystal structure 200, 201 forming a photonic band gap can function as an optical filter, which reflects or transmits light of at specific wavelength to amplify or reduce.

A photonic crystal structure 200, 201 is formed of the nanoparticles 140. As discussed above, the FIGS. 1C and 1D depict spherical nanoparticles, but the nanoparticles 140 may have various shapes, such as ovals, wedges or stripes. Also as discussed above, the photonic crystal structure 200, 201 formed of the nanoparticles 140 may have a face-centered cubic structure, a diamond-like structure, an inverse opal structure or the like.

As shown in FIG. 1C, the photonic crystal structure 200 may be formed of the nanoparticles 140 in the form of a self-assembled monolayer, spherical nanoparticles, for example, a polystyrene bead can be used. At this time, the diameter of nanoparticles 140 can be λ/2 (where λ is a wavelength of light emitted from fluorescent materials). In the case of a self-assembled monolayer, the distance between the centers of adjacent nanoparticles 140 is the same as the diameter of nanoparticles 140. Therefore, the diameter of nanoparticles 140 can be, for example, in the range of about 100 nm to about 1000 nm, preferably about 200 nm to about 300 nm.

As shown in FIG. 1D, the photonic crystal structure 201 may be formed by arranging the nanoparticles 140 at regular intervals. The distance between the centers of adjacent nanoparticles 140 can be λ/2 (where λ is a wavelength of light emitted from fluorescent materials). The distance between the centers of adjacent nanoparticles 140 can be, for example, about 100 nm to about 1000 nm, preferably about 200 nm to about 300 nm.

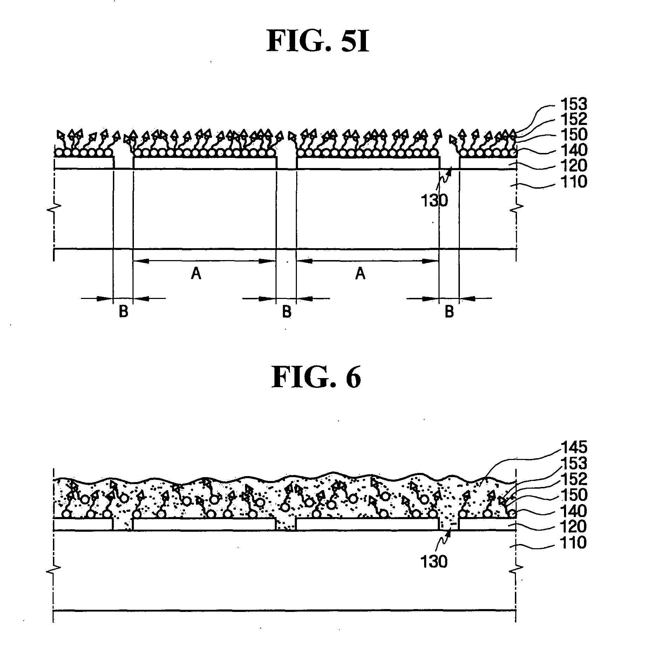

As shown in FIG. 5I, linkers 150 coupled to protective groups 153 are coupled to at least a portion of the nanoparticles 140 coupled to the surface of the probe cell region A. The linkers 150 may be formed from phosphoamidite, for example, in which the functional groups 152 of the linkers 150 are protected by protective groups 153.

The protective groups 153 prevent attachment sites from participating in a chemical reaction. Deprotecting means that the protective groups 153 are separated from the attachment sites, so that the sites do participate in the chemical reaction. For example, an acid labile or photo labile protective group 153 may be present in a functional group 152 coupled with a linker 150. The photo labile protective group 153 may be selected from various types of positive photo labile groups containing nitroaromatic compounds, such as o-nitrobenzyl derivatives or benzylsulfonyl. Examples of the photo labile protective group 153 may include 6-nitroveratryloxycarbonyl (NVOC), 2-nitrobenzyloxycarbonyl (NBOC), α, α-dimethyl-dimethoxybenzyloxycarbonyl (DDZ) or dimethoxytrityl (DMT). In this step, each protective group 153 is used to protecting a corresponding functional group 152 of a linker 150. However, the protective groups 153 may be used to protect a functional group of the nanoparticles 140 or the surface of an oligomer probe 165 immobilization layer.

After the coupling with the linkers 150, the functional groups 152 which are exposed on the surface and are not coupled with the linkers 150, are inactively capped using capping groups 155 to prevent the noise of the oligomer probe. Examples of the capping groups 155 include acetylation.

Subsequently, oligomer probes 165 are coupled to the probe cell region A, e.g., using in situ photolithography, to complete the oligomer probe array 100 as shown in FIG. 1B.

The specific region of FIG. 5I may be exposed to deprotect a photolabile group. A nucleotide phosphoamidite monomer coupled to the photolabile group is coupled to the exposed functional group. The functional group, which is not coupled with the monomer, is inactivated by capping, and subjected to oxidation in order to change phosphate trimester, generated by bonding between phosphoamidite and 5'-hydroxy group, to phosphate. That is, the process, which includes coupling a monomer with a desirable sequence and inactivating by capping the functional group, which is not coupled with the monomer, and subjecting the functional group to oxidation when a desirable probe cell region A is deprotected, is subsequently repeated to synthesize an oligonucleotide probe (e.g., probe 165) with a desirable sequence according to each probe cell region A.

Coupling of the oligomer probe 165 may be performed using a spotting method, a poexoelectric printing method, a micropipetting method, or the like, in addition to the photolithography, without departing from the spirit and scope of the present invention.

Another exemplary method of fabricating the oligomer probe array 100 according to the first embodiment is described with reference to FIG. 6. FIG. 6 is a cross-sectional view of a structure during a stage of the fabrication procedure of the oligomer probe array, according to the first embodiment of the present invention.

With reference to FIG. 6, a suspension 145 containing nanoparticles 140, which may be coupled with linkers 150 and protected by a photolabile group 153, is dispersed on the immobilization layer 120, as prepared in accordance with FIGS. 5A to 5E. The nanoparticles 140 are arranged on the probe cell region A of an immobilization layer 120 to form a photonic crystal structure. Subsequently, oligomer probe 165 are coupled in situ to fabricate the oligomer probe array 100 shown in FIG. 1B. The subsequent fabrication process steps are substantially the same as those described above with reference to FIGS. 5F to 5I.

In a method of fabricating the oligomer probe array, according to the second embodiment of the present invention, a photolabile group 153 and a linker 150 are coupled prior to coupling nanoparticles 140 with a probe cell region A. This method differs from the method of fabricating the oligomer probe array according to first embodiment, in which nanoparticles 140 are arranged to form photonic crystal structures 200, 201 and then linkers 150 are coupled. Therefore, damage in an edge region of the probe cell region A may be reduced.

With reference to FIGS. 5A to 5I, a method of fabricating the oligomer probe array 101 of FIG. 1F, according to the second embodiment of the present invention, is described.

The oligomer probe array 101 according to the second embodiment of the present invention can be formed by adjusting the concentration of the suspension 145 containing the nanoparticles 140. After forming a one-dimensional photonic crystal structure, as shown in FIG. 5H, a two-dimensional photonic crystal structure is formed by dispersing the suspension 145 containing the nanoparticles 140 to fabricate the oligomer probe array 101, as shown in FIG. 1F. A three-dimensional photonic crystal structure (not shown in the drawings) may be formed in the same manner, e.g., by again dispersing the suspension 145 containing the nanoparticles 140.

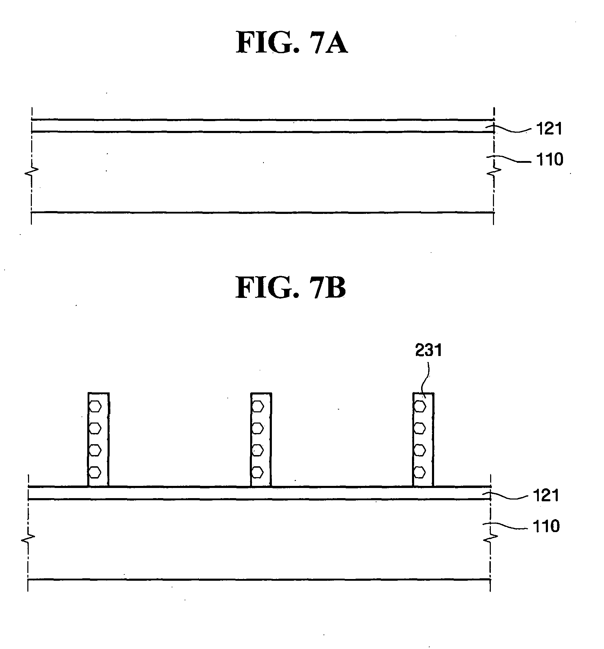

With reference to FIGS. 7A through 7D, a method of fabricating the oligomer probe array 102 of FIG. 2A, according to the third embodiment of the present invention, using a photoresist wall 231, is described.

FIGS. 7A to 7D are cross-sectional views of intermediate structures at various stages during a fabrication procedure of the oligomer probe array, according to the third embodiment of the present invention.

The fabrication method generally includes providing a substrate 110, forming an immobilization layer 121 on the substrate 110 and forming a photoresist wall 231 on at least a portion of the immobilization layer 121. Nanoparticles 140 are coupled to portions of the immobilization layer 121, on which the photoresist wall 231 is not formed, in order to form a photonic crystal structure. An oligomer probe 165 is coupled to the nanoparticles 140.

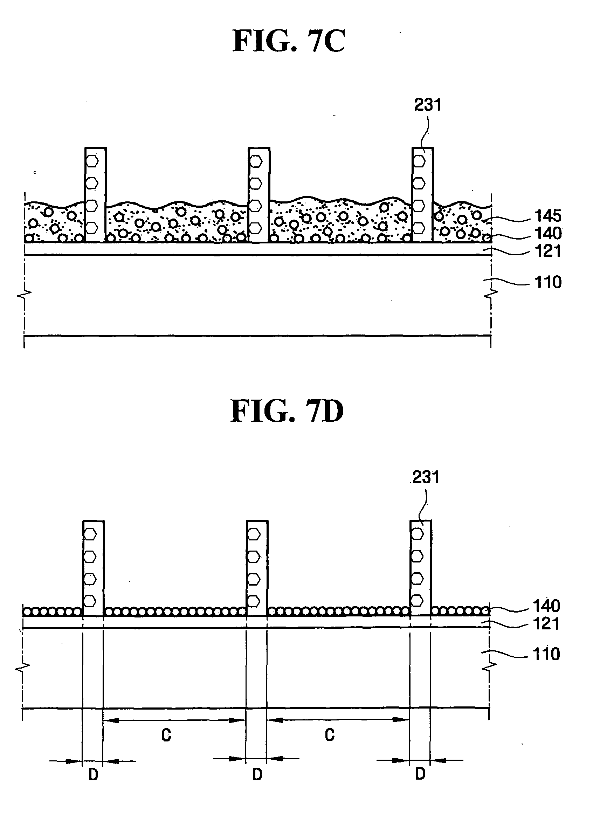

More particularly, as illustrated in FIG. 7A, a substrate 110 is provided and an immobilization layer 121 is formed on the substrate 110. As illustrated in FIG. 7B, a photoresist wall 231 is formed on at least a portion of the immobilization layer 122. As illustrated in FIG. 7C, a suspension 145 containing nanoparticles 140 is dispersed on the resultant structure of FIG. 7B.

After dispersing, as shown in FIG. 7D, the nanoparticles 140 are arranged on a probe cell region C, for example, in the form of a self-assembled monolayer on the surface of the probe cell region C. Through washing and curing, the nanoparticles 140 can be arranged only on the probe cell region C.

Subsequently, linkers 150 coupled with protective groups 153 are coupled with nanoparticles 140 that are coupled to the surface of the probe cell region C. The linkers 150 may be formed from phosphoamidite, for example, in which the functional group 152s of the 150 linkers are protected by protective groups 153.

The photoresist walls 231 make it relatively easier to couple the nanoparticles 140 with the immobilization layer 121. More specifically, upon arranging the nanoparticles 140, each photoresist wall 231 functions as a barrier, thereby making it easier to arrange the nanoparticles 140 regularly, e.g., to form nanoparticles in the form of a self-assembled monolayer. Nanoparticles 140 are not formed on portions of the immobilization layer 121 on which the photoresist walls 231 are formed. Therefore, a photonic crystal structure formed by the nanoparticles 140 cannot be generated. When photoresist walls 231 are used, there is no need of another patterning process for separating probe cell regions, coupled with nanoparticles 140, and probe cell separation regions, not coupled with the nanoparticles 140.

Subsequently, not shown in the drawings, after the linkers 150 are coupled with nanoparticles 140 to remove the photoresist walls 231, oligomer probes 165 are subjected to coupling using in situ photolithography, for example. The process is substantially the same as the method described with reference to FIGS. 5F to 6.

Also not shown in the drawings, when photoresist walls 231 are not used, the process, which includes forming a photoresist pattern on an immobilization layer 121, selectively capping the immobilization layer 121 exposed by a photoresist pattern, removing the photoresist pattern and then coupling nanoparticles 140, sequently proceeds to form an active region coupled with nanoparticles 140 and an inactive region being capped.

A method of fabricating the oligomer probe array 102 of FIG. 2A, according to the third embodiment of the present invention, using a photoresist wall 231 is described above. However, methods using photoresist walls 231 are not limited to this description. For example, the use of photoresist walls 231 may also be applied to the method of fabricating the oligomer probe arrays 100 and 101, according to the first and second embodiments, respectively. That is, after forming a probe cell region A by patterning an immobilization layer 120, a photoresist wall 231 may be formed on a probe cell separation region B to arrange nanoparticles 140.

Referring to FIG. 2B, a method of fabricating the oligomer probe array 103, according to the fourth embodiment of the present invention, is described.

Not shown in the drawings, the oligomer probe array 103, according to the fourth embodiment, can be formed by adjusting the concentration of a suspension 145 containing nanoparticles 140. Additionally, after forming a one-dimensional photonic crystal structure as shown in FIG. 7D, for example, a two-dimensional photonic crystal structure may be formed by dispersing a suspension 145 containing nanoparticles 140 to fabricate the oligomer probe array 103, as illustrated in FIG. 2B. Likewise, a three-dimensional photonic crystal structure (not shown in the drawings) can be formed in the same manner, by again dispersing the suspension 145 containing nanoparticles 140.

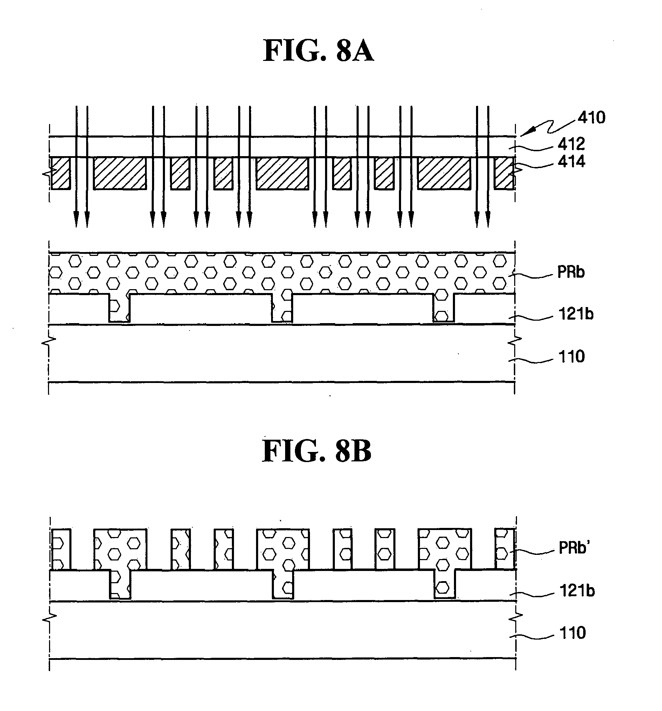

With reference to FIGS. 5A to 5I and FIGS. 8A to 8G, a method of fabricating the oligomer probe array 104, according to the fifth embodiment of the present invention, is described.

FIGS. 8A to 8G are cross-sectional views of intermediate structures at various stages of a procedure to fabricate the oligomer probe array according to the fifth embodiment of the present invention. The method includes forming the surface of a probe cell region A' of an oligomer probe array 104 three-dimensionally.

An immobilization layer 122b is patterned in substantially the same manner described with respect to the immobilization layer 120a of FIGS. 5A to 5E.

As illustrated in FIG. 8A, a photoresist layer PRb is applied on the resultant structure of FIG. 5E to expose the photoresist layer PRb using a photomask 410 defining a groove G pattern. Examples of the photomask 410 for defining the groove G pattern include a mask having a checkerboard pattern exposed region by a light shielding pattern 414, defining a probe cell region on a transparent substrate 412. However, the type of light shielding pattern may vary according to the type of photoresist layer being used.

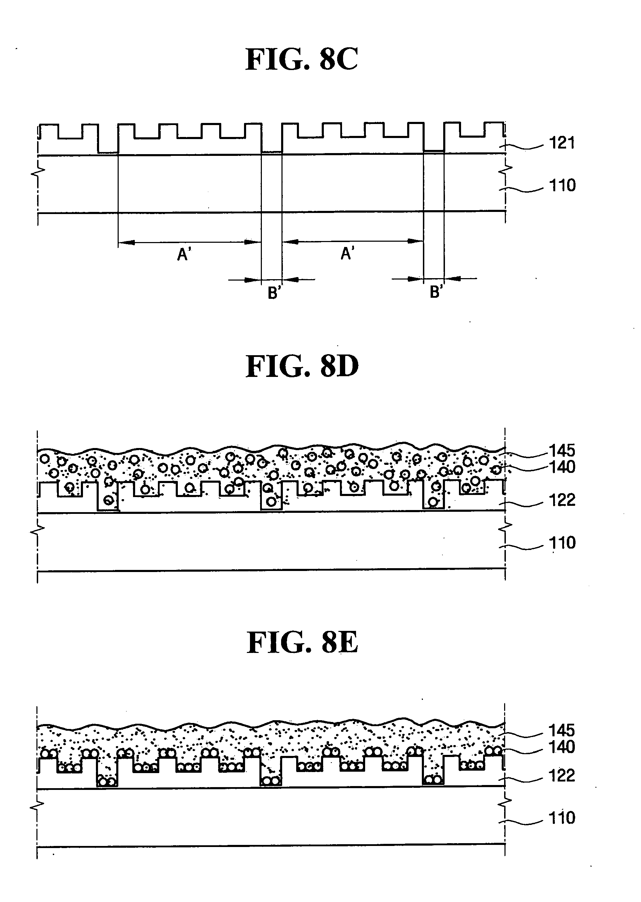

With reference to FIG. 8B, the exposed photoresist layer PRb is developed to form a photoresist pattern PRb' defining a groove G pattern. An etching process is performed using the photoresist pattern PRb' as an etch mask to obtain a probe cell region A', having the internal groove G of the immobilization layer 122, as shown in FIG. 8C.

As illustrated in FIG. 8D, a suspension 145 containing nanoparticles 140 is dispersed on the resultant structure of FIG. 8C. As shown in as shown in FIG. 8E, the nanoparticles 140 are arranged on the probe cell region A' following the dispersion, thereby forming a photonic crystal structure. The subsequent process is substantially the same as the method described with reference to FIGS. 5F to 6, above.

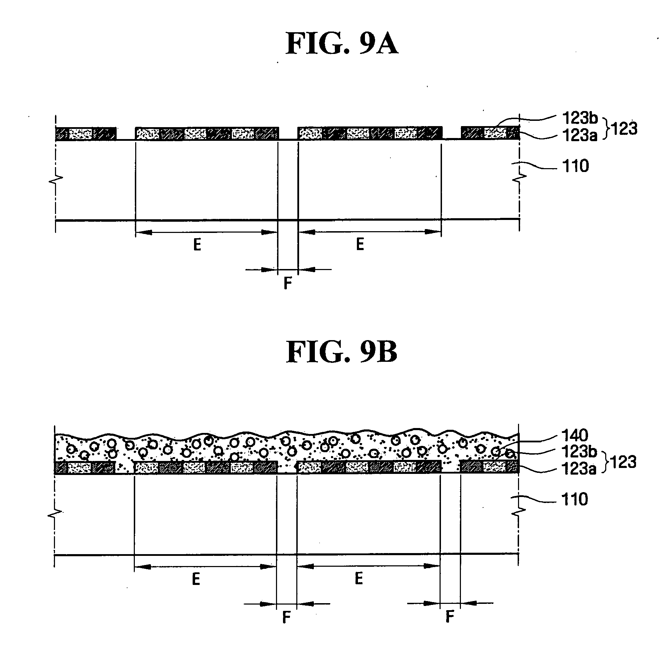

Next, with reference to FIGS. 4A and 4B and FIGS. 9A and 9B, the oligomer probe array 105, according to the sixth embodiment of the present invention, is described.

The oligomer probe array 105 according to the sixth embodiment has another photonic crystal structure 123, in addition to the photonic crystal structure formed by the nanoparticles 140. The photonic crystal structure 123 can be formed using an etching process, for example, which is a known semiconductor fabrication process, but the depicted embodiment is not limited to this process.

With reference to FIG. 9A, a method of forming a one-dimensional photonic crystal structure 123 is described. First, a substrate 110 is provided and a first dielectric 123a is formed on an entire surface of the substrate 110. In order to form the first dielectric 123a of a photonic crystal structure, a photoresist layer is formed and exposed to complete a photoresist pattern. The first dielectric 123a is etched according to the photoresist pattern. The method of etching the first dielectric 123a includes a wet etching and a dry etching, for example. To achieve a precise pattern, a dry etching can be used, such as an anisotropic etching. Widths of the first dielectric 123a and a second dielectric 123b forming a photonic crystal structure can be one forth of the light wavelength (λ) emitted from fluorescent materials 180. For example, the cycle of a photonic crystal structure 310 may be substantially half of the light wavelength (λ) emitted from the fluorescent materials 180. Subsequently, the residual photoresist pattern on the first dielectric 123a is removed to form a second dielectric 123b thereon. The second dielectric 123b formed on the first dielectric 123a is flattened by chemical mechanical polishing (CMP), for example, to form a photonic crystal structure 123, in which two dielectrics having different refractive indexes are arranged one-dimensionally and alternately.

As illustrated in FIG. 9B, a suspension 145 containing nanoparticles 140 is dispersed on the resultant structure. The subsequent process is substantially the same as the method described with reference to FIGS. 5F to 6.

A method of fabricating the oligomer probe array 106, according to a seventh embodiment of the present invention as shown in FIG. 4C, is described. When an immobilization layer 123 is a two-dimensional photonic crystal structure 123', the process illustrated in FIG. 9A is repeated to form a photonic crystal structure, on which multiple photonic crystal layers are disposed. As illustrated in FIG. 4C, a first dielectric 123a and a second dielectric 123b are alternately arranged in a parallel direction to each other on each layer, according to the seventh embodiment.

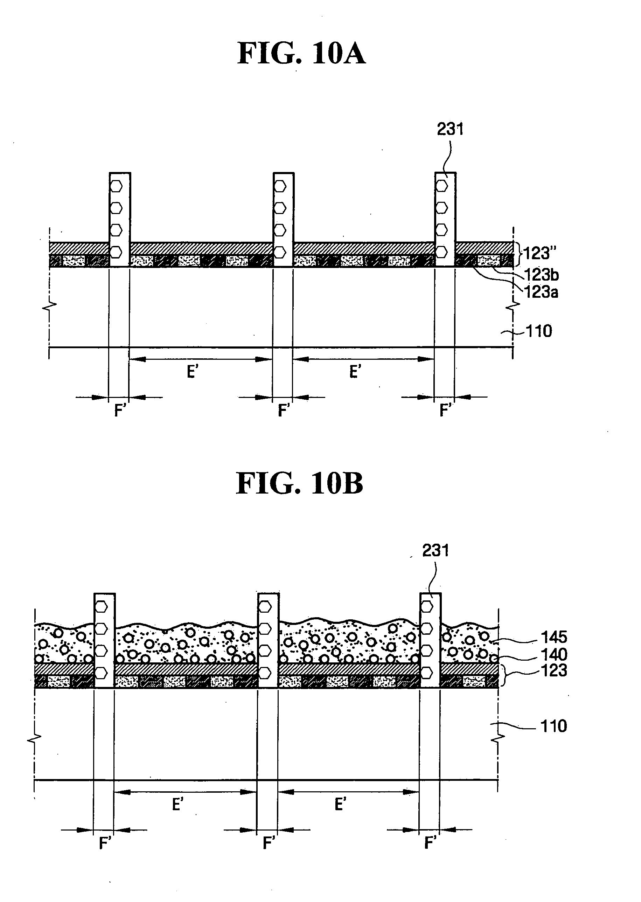

With reference to FIGS. 4D, 10A and 10B, a method of fabricating the oligomer probe array 107, according to an eighth embodiment of the present invention, is described. In order to form a three-dimensional photonic crystal structure 123", a layer-by-layer process based on a method, such as photolithography and an ion etching technique, can be used, although the type of process is not limited thereto. Also, FIGS. 10A and 10B include formation of a photoresist wall 231 on at least a portion of the immobilization layer 123", and dispersion of a suspension 145 containing nanoparticles 140, as shown in FIGS. 7B and 7C.

As discussed above, a one-dimensional photonic crystal layer is formed during the fabrication method shown in FIGS. 9A and 9B, for example, and a two-dimensional photonic crystal layer is formed by repeating the fabrication method shown in FIG 9A. A three-dimensional photonic crystal structure 123", shown in FIGS. 10A and 10B, is different from the two-dimensional photonic crystal structure 123', in that an n^{th} photonic crystal layer and an n+1^{st} photonic crystal layer have first dielectrics 123a that are arranged perpendicular to each other, and second dielectrics 123b, alternating between the first dielectrics 123a, that are arranged perpendicular to each other. Accordingly, a next photonic crystal layer is vertically patterned with respect to the preceding photonic crystal layer. Therefore, each dielectric 123a, 123b of each photonic crystal layer is vertically arranged with respect to each other and the same dielectrics of an n^{st} photonic crystal layer and an n+1^{st} photonic crystal layer can be alternately arranged with respect to each other.

Additional description related to the various embodiments of the present invention is provided below with reference to the specific experimental examples. Of course, additional examples analogous to the examples set forth herein are representative of the embodiments, although not specifically described.

### <EXAMPLE 1: Substrate modification>

In order to form nanoparticles only on the predetermined region of a silicon substrate, a thermal oxide pattern was formed on the silicon substrate using a photolithography process and an etching process. Reactive groups were attached on the surface of the thermal oxide pattern of the silicon substrate to react with aminosilane or hydroxysilane. Specifically, the silicon substrate was washed with a H₂SO₄ solution for 1 hour to activate a silanol of the surface. An aminosilane solution of 0.1% was applied on the silicon substrate and spun for 60 seconds at 50 rpm. The substrate was left at normal temperature for 14 minutes to volatilize the residual solvent slowly. Next, the substrate was baked for 40 minutes at 120°C to fully start silanation reaction. The baked substrate was washed with deionized water for 35 minutes. In order to remove the residual solution, the substrate was washed with acetonitrile for 3 minutes, and then subject to spin dry.

### <EXAMPLE 2: Preparation of nanoparticle suspension>

Nanoparticles, each of which has carboxylic acid on its surface and a size of 240 nm, were dispersed in acetonitrile using a sonication. The mixture of NPPOC-TEG-amine and DCC (1:1) was injected into the nanoparticle suspension, and then sonicated at normal temperature for 1 hour to start the binding reaction between amine and COOH on the surface of the nanoparticles. Acetonitrile in the nanoparticle suspension was exchanged with fresh acetonitrile three to five times to remove unreactive NPPOC-TEG-amine. Subsequently, a nanoparticle suspension modified with a NPPOC spacer, a photolabile group was obtained.

### <EXAMPLE 3: Formation of nanoparticle array on ,surface of substrate>

The nanoparticle suspension, of which a surface had a photolabile group prepared in EXAMPLE 2, was mixed with DCC (dicarboxy carboimidide) as an activator. The mixed suspension of the nanoparticles and the DCC was applied on the silicon substrate patterned with aminosilane prepared in EXAMPLE 1, and left at normal temperature for 1 hour to induce reaction between amine of the substrate and COOH on the surface of the nanoparticles. Acetonitrile was applied on the silicon substrate that reacted with nanoparticles to remove unreactive nanoparticles and DCC molecules, and was then spun at 100 rpm to remove the residual solvent.

### <EXAMPLE 4: Synthesis of oligonucleotide on substrate>

Oligonucleotide probes were synthesized in situ using photolithography on the substrate containing a probe cell region and a cell isolation region, prepared in EXAMPLE 1 to EXAMPLE 3. Specifically, acetonitrile was applied and left for 5 minutes so that the surface of the nanoparticle array activated with OH maintained the proper condition for an amidite coupling reaction. Next, the substrate was spun at 100 rpm to remove the acetonitrile. A nitroaromatic group of NPPOC was changed to a hydroxyl group by an i-line exposure. An ASML PAS 5500 100D stepper was used as an i-line exposure at the wavelength of 365 nm, and the exposure was performed using a chrome on quartz mask, which has a checkerboard pattern and has an opening size of 11 um, at 1000mJ/cm² for 1 minute. Subsequently, an acetonitrile solution (hexane activated with amidite:tetrazole=1:1) was treated at normal temperature and subjected to coupling. Next, a THF solution (Ac2O/py/methylimidazole= 1: 1: 1) and an iodine solution of 0.02M based on THF were treated to start capping and oxidation of the nucleotide being not coupled.

Processes, such as deprotecting, coupling, capping and oxidation, were repeated to synthesize oligonucleotide probes having different sequences from each other in each probe cell region.

### Measurement of fluorescent intensity

Target molecules were subjected to hybridization with the oligomer probe array synthesized through the processes described in EXAMPLE 1 through EXAMPLE 4. The fluorescent intensity of the hybridized probes was scanned using a CCD (ArrayWorx) or a fluorescent microscope. For the CCD scanning, the surface of hybridized DNA chip was loaded in the direction of a CCD light source and light with an excitation wavelength of fluorescent materials labeled to target molecules was radiated thereto to measure the intensity of fluorescent signals obtained by excitation of fluorescent materials.

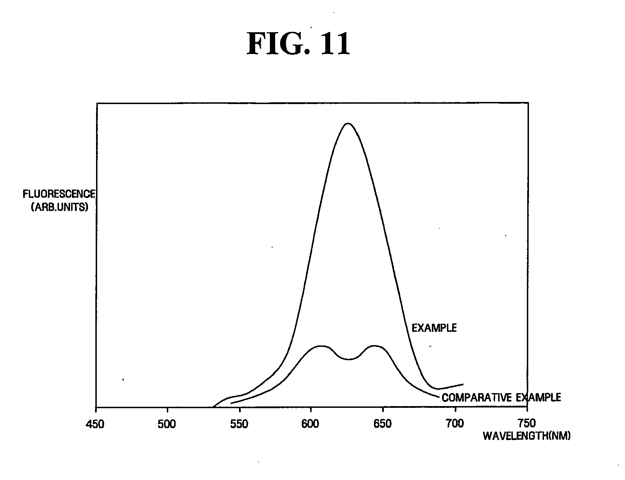

FIG. 11 is illustrates the result of a CCD measurement of the oligomer probe array, in which an oligonucleotide of 25mer was used as an oligomer probe.

With reference to FIG. 11, it was found that the amplification of fluorescent signals was improved in the oligomer probe array (EXAMPLE), in which a photonic crystal structure was formed by nanoparticles, as compared to oligomer probe array (COMPARATIVE EXAMPLE), in which a photonic crystal structure was not formed by nanoparticles.

### <EXAMPLE 5: Fabrication of probe cell region coupled with nanoparticles>

A siloxane resin, which had a thickness of 90 nm and contained the (CH₂)₁₀-OH group, was formed on a silicon substrate using the spin coating process and baked at 250°C for 60 seconds. A photoresist film having a thickness of 3.0 µm was formed on the substrate using the spin coating process, and then baked at 100°C for 60 seconds. The photoresist film was exposed in a projection exposing device having a wavelength of 365 nm using the dark tone of a checkerboard pattern mask having a pitch of 1.0 µm, and then developed with the 2.38% tetramethylammonium hydroxide aqueous solution to form the checkerboard photoresist pattern where the regions divided by lengths and widths of straight lines crossing with each other were exposed. The immobilization layer was etched using the photoresist pattern as the etching mask to perform patterning, thereby forming the probe cell region.

Ten ml of a suspension solution of silica beads (e.g., from Bangs Laboratory, Inc.), having a diameter of 490 nm and containing OH as the functional group, was dispersed on the substrate. Spin coating was then performed at 50 rpm for 60 seconds, washing was performed with IPA (isopropyl alcohol), and curing was performed at 110°C for 10 minutes.

### <EXAMPLE 6: Fabrication of probe cell region>

Siloxane, containing the (CH₂)₁₀- NH₂ group, was formed on a silicon substrate with a thickness of 90 nm using the spin coating process and baked at 250°C for 60 seconds. A photoresist film, containing the (CH₂)₁₀-NH₂ group and having a thickness of 3.0 µm, was formed on the substrate using the spin coating process, and then baked at 100°C for 60 seconds. The photoresist film was exposed in a projection exposing device having a wavelength of 365 nm using the dark tone of a checkerboard pattern mask having a pitch of 1.0 µm, and then developed with the 2.38% tetramethylammonium hydroxide aqueous solution to form the checkerboard photoresist pattern, where the regions divided by lengths and widths of straight lines crossing with each other were exposed. The immobilization layer was etched using the photoresist pattern as the etching mask to perform patterning, thereby forming the probe cell region.

Polystyrene beads (e.g., from Polyscience, Inc.), having the COOH group as the functional group and a diameter of 914 nm, were used as the nanoparticles. After the deionized suspension solution of 6.4 mmol of the nanoparticles, 3.1 mmol of 1-ethyl-3-(3-dimethylaminopropyl) carbodiimide, and 13.1 mmol of N-hydroxysuccinimide were dispersed, spin coupling was performed at 50 rpm for 60 seconds, washing was performed with IPA (isopropyl alcohol), and curing was performed at 110°C for 10 minutes.

An oligomer probe array according to the embodiments of the present invention immobilized nanoparticles on an immobilization layer to improve a reaction yield, as well as detecting sensitivity due to a hybridization reaction of a target molecule by a photonic crystal structure formed by nanoparticles. Thus, an oligomer probe array is provided which improves both reaction yield and detection sensitivity.

While the present invention has been described with reference to exemplary embodiments, it will be apparent to those skilled in the art that various changes and modifications may be made without departing from the spirit and scope of the present invention. Therefore, it should be understood that the above embodiments are not limiting, but illustrative.

## Claims

1. An oligomer probe array comprising:
a substrate;
an immobilization layer on the substrate;
a plurality of nanoparticles coupled to the immobilization layer, the plurality of nanoparticles forming a photonic crystal structure; and
a plurality of oligomer probes coupled to the plurality of nanoparticles,
wherein the immobilization layer comprises a plurality of probe cell regions coupled to the plurality of nanoparticles, adjacent probe cell regions being separated by a probe cell separation region.

2. The oligomer probe array of claim 1, wherein:
the nanoparticles provide a spatial margin for hybridization between the oligomer probes and target molecules; and
the photonic crystal structure amplifies a light wavelength emitted from a fluorescent material attached to the target molecules hybridized with the oligomer probes.

3. The oligomer probe array of claim 1, wherein each nanoparticle is spherical and has a diameter in the range of about 10 nm to about 1000 nm.

4. The oligomer probe array of claim 1, wherein a distance between centers of adjacent nanoparticles is in the range of about 100 nm to about 1000 nm

5. The oligomer probe array of claim 1, wherein each probe cell region comprises a three-dimensional surface.

6. The oligomer probe array of claim 1, wherein each probe cell region corresponds to the photonic crystal structure.

7. The oligomer probe array of claim 1, wherein the nanoparticles are arranged by self-assembly.

8. The oligomer probe array of claim 1, further comprising:
a plurality of linkers formed on surfaces of the plurality of nanoparticles, the plurality of nanoparticles being coupled to the plurality of oligomer probes through the corresponding plurality of linkers.

9. The oligomer probe array of claim 8, wherein a surface of each nanoparticle comprises a functional group configured to couple with one of the plurality of linkers.

10. The oligomer probe array of claim 1, wherein a surface of each nanoparticle comprises a functional group configured to couple with the immobilization layer and one of the plurality of oligomer probes.

11. The oligomer probe array of claim 1, wherein the plurality of nanoparticles are formed from at least one of spherical polystyrene, polymethylmethacrylate, polymethylmethacrylate copolymer, silica, glass, magnet, Wang resin, Merrifield resin, metal, plastic, cellulose, sephadex or sepharose.

12. The oligomer probe array of claim 1, wherein adjacent probe cell regions are physically separated by a probe cell separation region.
